# EUROPÄISCHE PATENTSCHRIFT

(11) **EP 0 727 205 B1**
(45) Veröffentlichungstag und Bekanntmachung des Hinweises auf die Patenterteilung: **30.05.2001**
(21) Anmeldenummer: 96101633.4
(22) Anmeldetag: 06.02.1996
(51) Int. Cl.: A61K 9/16, A61K 9/22

(54) **Thermoplastisches Überzugs- und Bindemittel für Arzneiformen**
Thermoplastic coating and binder agent for pharmaceutical forms
Agent thermoplastique d'enrobage et de liaison pour formes pharmaceutiques

(30) Priorität: 16.02.1995 DE 29502547 U
(43) Veröffentlichungstag der Anmeldung: 21.08.1996
(73) Patentinhaber: Röhm GmbH & Co. KG, 64293 Darmstadt (DE)
(72) Erfinder: Assmus, Manfred, D-64404 Bickenbach (DE); Petereit, Hans-Ulrich, D-64291 Darmstadt (DE)

(56) Entgegenhaltungen:
- EP-A- 0 040 590
- EP-A- 0 204 596
- EP-A- 0 502 642
- DE-A- 4 138 513
- US-A- 4 708 874
- US-A- 4 795 643
- US-A- 5 169 645

## Beschreibung

Die Erfindung betrifft ein thermoplastisches Überzugs- und Bindemittel für die Herstellung oder Umhüllung von Arzneiformen, insbesondere von festen, oral verabreichbaren Arzneiformen, aus dem schmelzflüssigen Zustand.

### Stand der Technik

Neben dem klassischen Verfahren zur Herstellung von festen Arzneiformen durch Pressen von Tabletten und gegebenenfalls Umhüllen mit einem in flüssiger Phase gelösten oder dispergierten Überzugsmittel gewinnt die Herstellung oder Umhüllung von Arzneiformen aus dem schmelzflüssigen Zustand des Überzugs- und Bindemittels als ein besonders wirtschaftliches und zuverlässiges Herstellungsverfahren zunehmende Bedeutung. Man benötigt dafür thermoplastische Überzugs- und Bindemittel, die eine Reihe von besonderen Anforderungen erfüllen müssen:
- Sie müssen die für die Anwendung der Arzneiform notwendigen Auflösungs- oder Freisetzungsanforderungen erfüllen.
- Sie müssen unzersetzt schmelzbar und durch Abkühlen in den festen Zustand versetzbar sein.
- Sie müssen beim Erstarren aus der Schmelze eine trockene, nicht klebrige Oberfläche ergeben.
- Sie dürfen unter den Bedingungen der thermoplastischen Verarbeitung den enthaltenen oder umhüllten pharmazeutischen Wirkstoff nicht schädigen.

Diese Forderungen werden durch die zur Zeit verfügbaren thermoplastischen Überzugs- und Bindemittel nicht in befriedigendem Maße erfüllt. Dies gilt vor allem für Überzugs- und Bindemittel auf Basis von Acryl-Kunststoffen, die unter dem Handelsnamen EUDRAGIT der Röhm GmbH, Darmstadt, allgemein bekannt sind. Sie zeichnen sich durch besondere Löslichkeits- und Freisetzungseigenschaften aus, die zur Herstellung von Retard-Präparaten unverzichtbar sind. So enthält EUDRAGIT E basische Aminogruppen, die die Löslichkeit im Magensaft gewährleisten. EUDRAGIT RL und RS enthalten quartäre Ammoniumgruppen, die die Wirkstoff-Freisetzung unabhängig vom pH-Wert des umgebenden wäßrigen Mediums steuern.

N. Follonier, E. Doelker und E. Cole (Drug development and Industrial Pharmacy, 20(8), 1323-1339, 1994) beschreiben die thermoplastische Verarbeitung von EUDRAGIT RS PM mit einem Zusatz von 3-5 % Triacetin als Weichmacher und Dilthiazem-HCl als Wirkstoff bei einer Temperatur von 110°C. Es wurden Arzneiformen mit makro- und mikroporöser Oberfläche erhalten, aus der der Wirkstoff mit deutlich höherer Geschwindigkeit freigesetzt wurde als aus Arzneiformen mit einem herkömmlichen Überzug von EUDRAGIT RS.

Die Herstellung von Arzneiformen durch Extrusion und Ausrundung unter Verwendung von thermoplastischen Überzugs- und Bindemitteln auf Basis von Acryl-Kunststoffen ist schon beschrieben worden. Soweit bei solchen Verfahren wäßrige Dispersionen oder feuchte Mischungen dieser Überzugs- und Bindemittel eingesetzt und ein Trocknungsschritt angewendet wird, entsprechen sie nicht der hier erörterten Zielsetzung der thermoplastischen Herstellung von Arzneiformen.

Gemäß DE-A 41 38 513 werden Gemische aus einem pharmazeutischen Wirkstoff und einem thermoplastischen Bindemittel aus der Schmelze extrudiert und zu Arzneiformen verarbeitet. Als thermoplastische Bindemittel werden Mischungen aus EUDRAGIT RL oder -RS mit Vinylpyrrolidon-Vinylacetat-Copolymeren und gegebenenfalls Hydroxypropylcellulose eingesetzt. Damit lassen sich nicht die gleichen Freisetzungseigenschaften erreichen wie mit den entsprechenden Acryl-Kunststoffen oder deren Mischungen allein.

Gemäß EP-A 204 596 werden wirkstoffhaltige Mikropartikel aus einer Mischung extrudiert, die als Bindemittel ein Polymer im Gemisch mit einem oder mehreren Exzipienten enthält. Die Exzipienten werden so gewählt, daß sie einzeln oder gemeinsam auf das Polymer eine lösende oder gelierende Wirkung sowie eine Gleitwirkung ausüben. Bei der Bearbeitung mittels perforierter Walzen erwärmt sich die Mischung, so daß einer der Exzipienten teilweise schmilzt und dabei seine lösende oder gelierende Wirkung entfaltet. Die auf diese Weise plastifizierte Mischung wird durch die Öffnungen der perforierten Walzen gepreßt und dabei zu Mikropartikeln extrudiert. Als Beispiel wird die Verarbeitung von pulverförmigem EUDRAGIT RS mit einem Glycerin-palmitostearat beschrieben.

Die Verarbeitung einer echten Schmelze dieser Mischung würde nicht zu brauchbaren Arzeiformen führen. Aus dem Polymer und dem Exzipienten würde eine homogene Mischung entstehen, die sich aufgrund der vorausgesetzten lösenden oder gelierenden Wirkung des Exzipienten beim Abkühlen nicht wieder entmischt. Die weichmachende Wirkung des Exzipienten hat zur Folge, daß die aus der Schmelze erstarrte Masse an ihrer Oberfläche weich und klebrig bleibt, so daß sie als Arneiformoberfläche nicht brauchbar wäre.

Ein thermoplastisches Überzugs- und Bindemittel auf Basis eines Acryl- und/oder Methacrylsäure-Methylacrylat-Copolymerisats ist aus dem deutschen Gebrauchsmuster G 94 14 065 bekannt. Es eignet sich zur Herstellung von darmsaftlöslichen Arznei-Umhüllungen aus der Schmelze bei Temperaturen von 120-180°C, nicht jedoch für die Zwecke, für die EUDRAGIT RL und -RS eingesetzt werden. Wegen seiner hohen Schmelztemperatur können Weichmacher zugesetzt werden, ohne daß die Oberfläche klebrig würde.

### Aufgabe und Lösung

Der Erfindung liegt die Aufgabe zugrunde, ein thermoplastisches Überzugs- und Bindemittel zur Verfügung zu stellen, das die eingangs genannten Forderungen erfüllt. Es soll eine Freisetzungs-Charakteristik erreicht werden, die derjenigen von herkömmlichen Filmüberzügen aus wäßrigen Dispersionen oder organischen Lösungen der gleichen Polymeren etwa entspricht. Voraussetzung dafür ist eine verbesserte Fließfähigkeit der Schmelze, jedoch ohne eine weichmachende Wirkung, die zu klebrigen Oberflächen führte.

Gegenstand der Erfindung ist ein thermoplastisches Überzugs- und Bindemittel für Arzneiformen, enthaltend eine inhomogene Mischung, die bei einer Mischungstemperatur von 100 - 150 °C gemischt wurde und aus der Schmelze erstarrt ist, aus:
A) 5 - 95 Gew.-%, bezogen auf die Gewichtssumme von A und B, eines thermoplastischen Acryl-Kunststoffes, **der ein Copolymerisat von Alkylestern der Acryl-und/oder Methacrylsäure und funktionellen Comonomeren mit kovalent gebundenen kationischen Gruppen ist,** mit einer Schmelztemperatur oberhalb Raumtemperatur, jedoch unter 200 °C, einer Glasübergangstemperatur unter 120 °C und einer Schmelzviskosität von 1000 bis 1.000.000 Pa · s bei der Schmelztemperatur und
B) 95 - 5 Gew.-% eines mit dem thermoplastischen Acryl-Kunststoff bei Raumtemperatur nicht verträglichen Fließmittels, das ein Fettalkohol, eine Fettsäure, ein Tensid, ein Fettsäure-mono-, -di- oder tri-glycerid, ein Polyethylenglykol, ein Zucker, ein Wachs, ein Fettsäure-Fettalkoholester, ein Zuckerester oder ein Polyethylenglykolfettsäureester oder ein Polyethylenglykolfettalkoholether ist, und eine Schmelztemperatur oberhalb Raumtemperatur, jedoch unter 200 °C, ein Molekulargewicht unter 20.000 Dalton und eine Schmelzviskosität unter 100 Pa s bei der Schmelztemperatur des Acryl-Kunststoffes hat.

Als Schmelztemperatur im Sinne der Erfindung gilt die niedrigste Temperatur, bei der die Schmelzviskosität, gemessen nach DIN 54811, den Grenzwert von 10⁶ Pa s unterschreitet. Die Glasübergangstemperatur wird thermisch mittels DSC (Differential Scanning Calorimetry) bestimmt. Als Molekulargewicht gilt der Gewichtsmittelwert, bestimmt durch Gel-Permeations-Chromatographie (GPC). Wenn eine entsprechende Eichkurve erstellt wird, ist die Bestimmung des Molekulargewichts aus der reduzierten Lösungsviskosität einfacher.

Die erfindungsgemäße Mischung aus den Bestandteilen A und B ist als inhomogene anzusehen, wenn die Komponenten A und B in den eingesetzen Mengen nicht verträglich sind. Dies ist an einem Film, der aus einer gemeinsamen Lösung der Komponenten in einem gemeinsamen Lösungsmittel, wie z.B. Aceton, beim Verdunsten des Lösungsmittels zurückbleibt, erkennbar. Ist der Film deutlich trüb, so läßt dies auf das Vorhandensein zweier miteinander nicht verträglicher Phase schließen. Bei begrenzter Verträglichkeit können sich zwei Phasen bilden, in denen jeweils eine der Komponenten anteilmäßig überwiegt. Solche Mischungen gelten im Sinne der Erfindung als unverträglich, wenn die Glasübergangstemperatur der Mischung nicht wesentlich, insbesondere nicht mehr als 20 K, unter der Glasübergangstemperatur des Polymeren A liegt.

Die Unverträglichkeit hat die Wirkung, daß in der erstarrten Schmelze die Komponenten A und B als getrennte Phasen vorliegen und das Fließmittel B nicht als Weichmacher in der Polymerphase A gelöst vorliegt. Die Phasen können in Domänen geringer Größe vorliegen; vorzugsweise bildet die Komponente B getrennte, disperse Phasen von 0,1 bis 500 Mikrometer Größe innerhalb der zusammenhängenden Phase A. Das Mengenverhältnis der Phasen A : B wird vorzugsweise so gewählt, daß A eine zusammenhängende Phase bildet. Die Phasengröße ist im licht- bzw. elektronenmikroskopischen Bild erkennbar.

Das Vorhandensein zweier Phasen läßt sich auch durch Differential-Thermoanalyse (DSC-Messung) nachweisen. Unverträgliche Mischungen zeichnen sich durch zwei thermische Signale aus, die im wesentlichen denen der Komponenten A und B entsprechen.

Im Schmelzezustand sind die Komponenten A und B im wesentlichen mischbar, erkennbar an der optischen Klarheit der Schmelze. Aus der Verträglichkeit im Schmelzezustand ergibt sich eine gute Fließfähigkeit und eine Schmelzviskosität von weniger als 500 Pa s, vorzugsweise 1 bis 200 Pa s. Dadurch werden eine schnelle Plastifizierung, eine niedrige Verarbeitungstemperatur, niedrige Scherkräfte bei der Verarbeitung, vorbunden mit einer geringen thermischen Zersetzung, eine vollständige und genaue Füllung von Formwerkzeugen und eine geschlossene, porenarme Oberfläche der erstarrten Schmelze erreicht. Das Freisetzungsverhalten wird hauptsächlich durch die Eigenschaften der Polymerisatkomponente A bestimmt und entspricht weitgehend demjenigen einer entsprechenden Arzneiform mit einer aus organischer Lösung des gleichen Polymeren A erzeugten Hülle.

### Ausführung der Erfindung

Unter thermoplastischen Acryl-Kunststoffen werden unvernetzte, allenfalls verzweigte, jedenfalls in geeigneten organischen Lösungsmitteln, wie Aceton, Isopropylalkohol oder Ethanol, zumindest kolloidal lösliche Polymerisate und Copolymerisate verstanden, die zu wenigstens 20 Gew.-%, vorzugsweise wenigstens 50 Gew.-%, aus Acrylmonomeren aufgebaut sind. Diese Monomeren zeichnen sich durch die Gruppe

CH₂=CR-CO-

aus, worin R entweder ein Wasserstoffatom oder eine Methylgruppe darstellt. Ihr Molekulargewicht beträgt vorzugsweise 10.000 bis 200.000 Dalton, ihre Schmelzviskosität bei 100°C vorzugsweise 10.000 bis 500.000 Pa s. Von wesentlicher Bedeutung ist eine Glasübergangstemperatur oberhalb der Raumtemperatur und unter 120°C; der bevorzugte Bereich ist 30 bis 80°C.

Die thermoplastischen Acryl-Kunststoffe sind im allgemeinen Copolymerisate von Estern der Acryl- und/oder Methacrylsäure, insbesondere Copolymerisate von Alkylestern der Acryl-und/oder Methacrylsäure und funktionellen Comonomeren mit kovalent gebundenen kationischen Gruppen. Vorzugsweise machen die Alkylester 5 bis 99 Gew.-% und die kationischen Comonomeren 1 bis 95 Gew.-% des Copolymerisats aus. Weitere Comonomere können gewünschtenfalls mitverwendet werden; als Beispiele sind Hydroxyalkylester oder Alkoxyalkylester der Acryl- und/oder Methacrylsäure oder kleine Mengen dieser Säure selbst, Abkömmlinge der Maleinsäure, Styrol, Vinylacetat zu nennen. Sie machen in der Regel nicht mehr als 40 Gew.-% des Copolymerisats aus.

Die Acrylester der Acryl- und/oder Methacrylsäure leiten sich vorzugsweise von niederen Alkanolen ab, insbesondere solchen mit 1 bis 4 Kohlenstoffatomen im Alkylrest. Methylacrylat und -methacrylat und Ethylacrylat und -methacrylat sind besonders bevorzugt.

Bei den kationischen Comonomeren kann es sich um Aminoalkylester oder Aminoalkylamide der Acryl- und/oder Methacrylsäure oder deren Salze oder Quaternierungsprodukte handeln. Sie enthalten vorzugsweise tertiäre Amino- oder quartäre Ammoniumgruppen, die über einen niederen Alkylenrest, vorzugsweise mit 1 bis 5 Kohlenstoffatomen, an das Ester-Sauerstoffatom oder das Amid-Stickstoffatom gebunden sind. Sie lassen sich in der Regel durch die Formel

CH₂=CR-CO-X-Alk-N(R')₂

oder

CH₂=CR-CO-X-Alk-N⁺(R')₃ An⁻

darstellen, worin R = H oder CH₃-, R' = C₁₋₄-Alkyl, X die Gruppe -O- oder -NH-, Alk einen geradkettigen oder verzweigten Aklylenrest mit 1 bis 5 Kohlenstoffatomen und An⁻ ein einwertiges Säureanion bedeuten.

Geeignete kationische Comonomere sind z.B.
2-(N,N-Dimethylamino-ethyl-acrylat und -methacrylat
3-(N,N-Dimethylamino)-propyl-acrylat und -methacrylat,
4-(N,N-Dimethylamino)-butyl-acrylat und -methacrylat,
3-(N,N-Dimethylamino)-propyl-acrylamid und -methacrylamid,
Triethanolamin-monoacrylat und -monomethacrylat,
2-(Dimethylaminoethyloxy)-ethyl-acrylat und -methacrylat,
2-Imidazolyl-ethyl-acrylat und -methacrylat,
2-Piperazinyl-ethyl-acrylat und -methacrylat,
2-Piperazinyl-ethyl-acrylamid und -methacrylamid,
N,N-Dimethylamino-neopentyl-acrylat und -methacrylat,
N,N-Dimethylamino-neopentyl-acrylamid und -methacrylamid,
(1,2,2,6,6-Pentamethyl-piperidyl-4)-acrylat und -methacrylat
3-Morpholino-propyl-acrylamid und -methacrylamid,
2-Morpholino-ethyl-acrylat und -methacrylat,
2-(N,N-Dibutylamino)-ethyl-acrylat und -methacrylat,
4-Diethylamino-1-methyl-butyl-acrylamid und -methacrylamid,
sowie die mit Methylchlorid oder anderen Quaternierungsmitteln daraus herstellbaren Quaternierungsprodukte und die Salze der genannten Monomeren mit organischen oder anorganischen Säuren, wie Salzsäure, Schwefelsäure, Phosphorsäure, Essigsäure, Propionsäure, Salicylsäure, Bernsteinsäure, Laurinsäure usw.

In der Praxis spielen Copolymerisate von Methylacrylat und-methacrylat und/oder Ethylacrylat und -methacrylat mit 1 - 95 Gew.-% Dimethylaminoethylacrylat und -methacrylat oder Trimethylammonioethylacrylat-chlorid und -methacrylat-chlorid die wichtigste Rolle. Zu dieser Gruppe gehören EUDRAGIT RL, -RS und -E.

Die erfindungsgemäß eingesetzten Polymere müssen eine der pharmazeutischen Verwendung angemessene Reinheit besitzen. Insbesondere der Restmonomerengehalt sollte unter 1000 ppm liegen.

Als Fließmittel B kommen Stoffe in Betracht, die sich mit der Schmelze des Polymeren A im wesentlichen homogen mischen lassen und die Fließfähigkeit der Schmelze verbessern, sich aber beim Abkühlen und Erstarren der Schmelze als eigene Phase abtrennen. Stoffe, die diese Voraussetzungen erfüllen, haben ein deutlich niedrigeres Molekulargewicht als das Polymer A und eine davon abweichende, im allgemeinen niedrigere Polarität. Die Polarität wird durch den Anteil hydrophiler und hydrophober Gruppierungen in der Molekülstruktur des Fließmittels bestimmt. Sauerstoffatome in Form von Hydroxyl-, Ether-, Ester- und Carbonylgruppen sowie Stickstoffatome in Form von Amino-, Ammonium- und Amidgruppen, die jeweils auch in cyclischen Molekülstrukturen vorkommen können, erhöhen die Polarität. Dagegen wird die Polarität durch längere aliphatische oder olefinische Reste, besonders solche mit 6 bis 30 Kohlenstoffatomen, und durch aromatische Reste herabgesetzt.

Liegt die Polarität des Fließmittels zu hoch, so überwiegen seine lösenden und weichmachenden Eigenschaften. Sie verhindern die Phasentrennung beim Erstarren und führen zu einer klebrigen Oberfläche. Liegt die Polarität zu niedrig, besteht die Gefahr, daß das Gemisch aus A und B auch in der Schmelze zweiphasig bleibt. Dann kann die erwünschte Verbesserung der Fließfähigkeit ausbleiben oder es kommt sogar zu Entmischungsvorgängen, die bei der Extrusion sehr störend sein können. Es ist einfacher, die Verträglichkeit im Schmelzezustand und die Unverträglichkeit im erstarrten Zustand durch Vorversuche zu ermitteln als sie anhand von Polaritätsinkrementen zu berechnen. Die Wahl eines geeigneten Fließmittels kann manchmal erleichtert werden, wenn man Mischungen verschiedener Stoffe, insbesondere solcher von unterschiedlicher Polarität, herstellt und die bestgeeignete Gesamtpolarität durch Variation des Mischungsverhältnisses ermittelt.

Die Verträglichkeitseigenschaften werden jedoch auch durch das Molekulargewicht des Fließmittels beeinflußt. Im allgemeinen nimmt die Verträglichkeit - bei sonst gleichbleibender Molekülstruktur -mit steigendem Molekulargewicht ab. So sind z.B. Polyethylenglykole mit Molekulargewichten unter 1000 Dalton als Fließmittel meistens weniger geeignet als solche mit Molekulargewichten von 1000 bis 20 000 Dalton. Bei den etwas weniger polaren Polypropylenglykolen liegt die Molekulargewichtsgrenze niedriger. In der Regel sind Stoffe mit Molekulargewichten über 20 000 Dalton ungeeignet, da sie die Schmelzviskosität nicht im gewünschten Ausmaß vermindern.

Geeignete Fließmittel finden sich in unterschiedlichen Stoffklassen, z.B. unter Fettalkohole, Fettsäuren, Tensiden, Mono-, Di- und Triglyceride. Wichtig ist eine Schmelztemperatur oberhalb Raumtemperatur, aber unter 200°C, vorzugsweise im Bereich von 30 bis 150, insbesondere 40 bis 80°C.

Für Copolymerisate von niederen Alkylacrylaten und-methacrylaten mit 5 bis 10 Gew.-% an kationischen, Amino- bzw. Ammoniumgruppen enthaltenden Comonomeren vom Typ EUDRAGIT RL, -RS und -E haben sich Fettsäuremonoglyceride, insbesondere Glycerinmonostearat, und Polyethylenglykole mit Molekulargewichten von 4000 bis 8000 Dalton als gut geeignet erwiesen.

Als Beispiele weiterer Fließmittel sind zu nennen:
Fettsäuren, wie z.B.
   Stearinsäure,
   Laurinsäure,
   Palmitinsäure,
   Caprinsäure,
   Myristinsäure,
Zucker, wie z.B.
   Sorbit
Wachse, wie z.B.
   Bienenwachs
   Wollwachs,
   Walrat,
Fettalkohole, wie z.B.
   Cetylalkohol
   Stearylalkohol
Polyethylenglycole, wie z.B.
   PEG 1000
   PEG 2000
   PEG 6000
Glycerinester, wie z.B.
   Glycerinlaurat,
   Glycerinmonostearat,
   Glycerindistearat,
   Glycerintristearat
Fettsäure-Fettalkohol-Ester, wie z.B.
   Palmitinsäurecetylester
Zuckerester, z.B.
   Sorbitanmonostearat,
Polyethylenglykolfettsäureester, z.B.
   Polyethylenglycol-400-stearat
Polyethylenglykolfettalkoholäther, z.B.
   Polyethylenglykol-23-lauryläther.

Ebenso sind Mischung von Fließmitteln der genannten Stoffklassen geeignet, sofern sie als Mischung die für das Fließmittel geforderten Eigenschaften aufweisen.

Die Menge des Fließmittels bzw. das Mischungsverhältnis A : B richtet sich nach der fließverbessernden Wirksamkeit und somit auch nach den Anforderungen des Verarbeitungsverfahrens. Im allgemeinen wird eine Schmelzviskosität des thermoplastisches Überzugs- und Bindemittels von weniger als 4000 Pa s, vorzugsweise unter 1000 Pa s, insbesondere unter 500 Pa s angestrebt, jeweils gemessen bei 100°C. In vielen Fällen sind Schmelzviskositäten von 50 bis 250 Pa s erreichbar. Der Anteil des Fließmittels liegt dann in der Regel zwischen 10 und 60 Gew.-%, vorzugsweise 20 bis 50 Gew.-%, bezogen auf die Gewichtssumme von A und B.

Ein Einfluß des Fließmittels auf die Freisetzungseigenschaften im Vergleich zu der reinen Polymerkomponente A ist nicht auszuschließen und muß gegebenenfalls bei der Zusammenstellung der Mischung berücksichtigt werden. So können Carbonsäuren als Fließmittel die Auflösung oder Permeabiltät des thermoplastischen Überzugs- und Bindemittels im Darmsaft bei steigenden pH-Wert fördern.

Die erfindungsgemäßen thermoplastischen Überzugs- und Bindemittel werden zweckmäßig im gewünschten Mischungsverhältnis vorgefertigt. Dazu werden die Komponenten A und B und gewünschtenfalls weitere in Arzneimittelüberzügen gebräuchliche Zusätze, wie Füllstoffe, Pigmente, Farbstoffe, Dispergiermittel, Stabilisatoren, Aromastoffe in der Schmelze gleichförmig gemischt, abgekühlt und nach dem Erstarren zu einem Pulver oder Granulat zerkleinert. Insbesondere können anionische Polymere, die unter den angegebenen Bedingungen nicht schmelzen zur Modifizierung des Freigabeverhaltens eingesetzt werden. Mit Vorteil wird ein Mischextruder verwendet, wobei vorzugsweise die Polymerisatkomponente A als Pulver oder Granulat als Mischung mit dem Fließmittel B eingebracht, aufgeschmolzen und homogenisiert wird. Die Mischungstemperatur liegt z.B. bei 100 bis 150°C. Die Mischung wird als Strang aus dem Extruder ausgetragen und durch Heißabschlag oder durch Brechen nach dem Erkalten granuliert. Auch heizbare Kneter sind verwendbar.

Die im Sinne der Erfindung eingesetzen Arzneistoffe sind dazu bestimmt, am oder im menschlichen oder tierischen Körper Anwendung zu finden, um
1. Krankheiten, Leiden, Körperschäden oder krankhafte Beschwerden zu heilen, zu lindern, zu verhüten oder zu erkennen,
2. die Beschaffenheit, den Zustand oder die Funktionen des Körpers oder seelische Zustände erkennen zu lassen,
3. vom menschlichen oder tierischen Körper erzeugte Wirkstoffe oder Körperflüssigkeiten zu ersetzen,
4. Krankheitserreger, Parasiten oder körperfremde Stoffe abzuwehren, zu beseitigen oder unschädlich zu machen oder
5. die Beschaffenheit, den Zustand oder die Funktionen des Körpers oder seelische Zustände zu beeinflussen.

Gebräuchliche Arzneistoffe sind Nachschlagewerken, wie zB. der Roten Liste oder dem Merck Index zu entnehmen. Erfindungsgemäß können alle Wirkstoffe eingesetzt werden, die die gewünschte therapeutische Wirkung im Sinne der obigen Definition erfüllen und eine ausreichende thermische Stabilität besitzen. Wichtige Beispiele (Gruppen und Einzelsubstanzen) sind folgende:
Antiallergika, Antiarrhythmika, Antibiotika/Chemotherapeutika, Antidiabetika, Antiepileptika, Antihypertonika, Antihypotonika, Antikoagulantia, Antimykotika, Antiphlogistika, Betarezeptorenblocker, Calciumantagonisten und ACE-Hemmer, Broncholytika/Antiasthmatika, Corticiode (Interna), Dermatika, Diuretika, Enzyminhibitoren, Enzympräparate und Transportproteine, Geriatrika, Gichtmittel, Grippemittel, Hypnotika/Sedativa, Kardiaka, Lipidsenker, Nebenschilddrüsenhormone/Calciumstoffwechselregulatoren, Psychopharmaka, Sexualhormone und ihre Hemmstoffe, Spasmolytika, Wundbehandlungsmittel, Zytostatika.

Für größere Produktionen von Arzneiformen kann es zweckmäßig sein, schon bei der Herstellung der Mischung in der Wärme einen oder mehrere pharmazeutische Wirkstoffe in die Mischung einzuarbeiten. Das erhaltene Granulat kann dann unmittelbar in Kapseln abgefüllt oder gegebenenfalls unter Zusatz von Tablettierhilfsstoffen, zu Tabletten verpreßt werden.

Verfahren zur Herstellung fester Arzneiformen auf thermoplastischem Wege sind bekannt. Dazu gehören Spritzgießverfahren, die allerdings lange Zeit als wenig geeignet angesehen wurden, weil bei der Herstellung spritzgegossener Tabletten Angußkörper anfallen, die wegen ihres Gehaltes an wertvollen Wirkstoffen nicht einfach verworfen oder entsorgt werden konnten. Gegen die erneute thermoplastische Verarbeitung bestanden Bedenken, weil mit unkontrollierbaren Zersetzungen unter hohen Verarbeitungstemperaturen und Scherkräften gerechnet wurde. Durch die Erfindung werden nun ziemlich niedrige Verarbeitungstemperaturen und eine hohe Fließfähigkeit ermöglicht, so daß die thermische und rheologische Belastung des Materials begrenzt ist und Angußkörper von geringem Volumen ausreichend sind. Es sollte daher im Einzelfall geprüft werden, ob die Tablettenherstellung im Spritzgießverfahren vertretbar ist.

Im allgemeinen wird die Herstellung von Arneiformen durch Extrusion eines Stranges bevorzugt. Durch Heißabschlag lassen sich Dosiseinheiten abtrennen, die noch vor dem Erstarren mit mechanischen Mitteln oder im Warmluftwirbelstrom ausgerundet werden können.

Flächige Arzneiformen, wie z.B. transdermal wirkende Pflaster, lassen sich durch Extrusion von Folien, gegebenenfalls auf flächigen Trägern, herstellen.

Wirkstofffreie Überzugsmassen können zum Umspritzen von gepreßten Tabletten eingesetzt werden. Diese können zu Beginn des Spritzvorganges mittels Stützstempeln im Zentrum eines Formhohlraumes gehalten und von der Schmelze eingeschlossen werden. Vor dem Erstarren werden die Stützstempel zurückgezogen und die Umhüllung des Tablettenkerns vervollständigt.

Niedrigviskose Schmelzen lassen sich in Zwangsmischern oder Wirbelbettanlagen auf Tabletten- und Drageekerne aufsprühen. Zweckmäßig läßt man die beschichteten Arzneiformen aus einem Warmluftwirbelbett zur Abkühlung in ein Kaltluftwirbelbett übertreten, aus dem die überzogenen Arzneiformen abgenommen werden. Es können übliche Schichtdicken, beispielsweise 10 bis 200 Mikrometer, erreicht werden. Ein geeignetes Beschichtungsverfahren wurde von M.J. Jozwiakowski et al. in Pharmaceutical Research, Vol.7, Nov.1990, S.3-10, beschrieben.

In der Regel zeigen die gemäß der Erfindung hergestellten Arzneiformen das für die Polymerisatkomponente typische Freisetzungsverhalten des enthaltenen Wirkstoffes in den Magen- oder Darmsaft. Arzneiformen mit einer Matrix oder einem Überzug auf Basis eines Aminogruppen enthaltenden Polymeren, wie EUDRAGIT E, lösen sich in künstlichem Magensaft innerhalb maximal 2 Stunden auf und geben den Wirkstoff frei. Polymere mit quartären Ammoniumgruppen, wie EUDRAGIT RL oder -RS, ergeben Überzüge oder Matrices, deren Löslichkeit und Diffusionsdurchlässigkeit vom pH-Wert des umgebenden wäßrigen Mediums unabhängig ist. Die Arzneiformen bleiben in künstlichem Magen- und Darmsaft ungelöst und geben den Wirkstoff durch Diffusion in Abhängigkeit vom Ammoniumgruppengehalt allmählich frei. Durch Abmischung von EUDRAGIT RL und RS können Zwischenwerte der Freisetzungsgeschwindigkeit eingestellt werden.

### BEISPIELE

1. 500 g EUDRAGIT RS 100 werden in einem heizbaren Kneter mit 250 g Glycerolmonostearat bei 120 Grad C gemischt. Die entstandene Masse ist bei Raumtemperatur weißlich gefärbt, fest und homogen.
   Die Glasübergangstemperatur (DSC) liegt bei 50 Grad C, die Schmelzviskosität bei 100 Grad C unter 100 Pas.
2. 500 g EUDRAGIT RS 100 werden in einem heizbaren Kneter mit 250 g Polyethylenglykol 6000 bei 120 Grad C gemischt. Die entstandene Masse ist bei Raumtemperatur weißlich gefärbt, fest und homogen.
   Die Glasübergangstemperatur (DSC) liegt bei 50 Grad C, die Schmelzviskosität beträgt bei 100 Grad C bei 221 Pas.
3. 500 g EUDRAGIT RL 100 werden in einem heizbaren Kneter mit 250 g Raumtemperatur 6000 bei 120 Grad C gemischt. Die entstandene Masse ist bei Raumtemperatur weißlich gefärbt, fest und homogen.
   Die Glasübergangstemperatur (DSC) liegt bei 55 Grad C, die Schmelzviskosität bei 100 Grad C und beträgt 2858 Pas.
4. 400 g EUDRAGIT E 100 werden in einem heizbaren Kneter mit 300 g Glycerolmonostearat bei 120 Grad C gemischt. Die entstandene Masse ist bei Raumtemperatur weißlich gefärbt, fest und homogen.
   Die Glasübergangstemperatur (DSC) liegt bei 50 Grad C, die Schmelzviskosität unter 100 Grad C unter 100 Pas.
5. In 350 g der unter 4. hergestellten Masse wird in 35 g Methionin eingearbeitet. Die entstandene Masse ist bei Raumtemperatur weißlich gefärbt, fest und homogen. Die Glasübergangstemperatur liegt bei ca. 50 Grad C, die Schmelzviskosität bei 100 Grad C unter 100 Pas. Das Freigabeverhalten des Wirkstoffs ist von dem Polymeren gesteuert.

## Patentansprüche

1. Thermoplastisches Überzugs- und Bindemittel für Arzneiformen, enthaltend eine inhomogene Mischung, die bei einer Mischungstemperatur von 100 - 150 °C gemischt wurde und aus der Schmelze erstarrt ist, aus:
A) 5 - 95 Gew.-%, bezogen auf die Gewichtssumme von A und B, eines thermoplastischen Acryl-Kunststoffes, der ein Copolymerisat von Alkylestern der Acryl-und/oder Methacrylsäure und funktionellen Comonomeren mit kovalent gebundenen kationischen Gruppen ist, mit einer Schmelztemperatur oberhalb Raumtemperatur, jedoch unter 200 °C, einer Glasübergangstemperatur unter 120 °C und einer Schmelzviskosität von 1000 bis 1.000.000 Pa · s bei der Schmelztemperatur und
B) 95 - 5 Gew.-% eines mit dem thermoplastischen Acryl-Kunststoff bei Raumtemperatur nicht verträglichen Fließmittels, das ein Fettalkohol, eine Fettsäure, ein Tensid, ein Fettsäure-mono-, -di- oder tri-glycerid, ein Polyethylenglykol, ein Zucker, ein Wachs, ein Fettsäure-Fettalkoholester, ein Zuckerester oder ein Polyethylenglykolfettsäureester oder ein Polyethylenglykolfettalkoholether ist, und eine Schmelztemperatur oberhalb Raumtemperatur, jedoch unter 200 °C, ein Molekulargewicht unter 20.000 Dalton und eine Schmelzviskosität unter 100 Pa·s bei der Schmelztemperatur des Acryl-Kunststoffes hat.

2. Thermoplastisches Überzugs- und Bindemittel nach Anspruch 1, dadurch gekennzeichnet, daß der thermoplastische Acryl-Kunststoff A ein Copolymerisat von 5 bis 99 Gew.-% Alkylestern der Acryl-und/oder Methacrylsäure und 1 bis 95 Gew.-% Aminoalkylestern oder Aminoalkylamiden der Acryl- und/oder Methacrylsäure oder deren Salzen oder Quaternierungsprodukten ist.

## Claims

1. A thermoplastic coating and bonding agent for medicinal forms, containing an inhomogenous mixture which is mixed at a mixing temperature of 100 - 150° C and solidifies from the melt, comprising:
a) 5 to 95 wt.-%, based on the sum of the weights of A and B, of a thermoplastic acrylic plastic which is a copolymerisate of alkyl esters of acrylic and/or methacrylic acid and functional comonomers with covalently-bonded cationic groups, with a melt temperature above room temperature but below 200° C, a glass transition temperature of less than 120° C and a melt viscosity of 1000 to 1 000 000 Pa·s at melt temperature, and
b) 95 to 5 wt.-% of a flow agent which is not compatible with the thermoplastic acrylic plastic at room temperature, this being a fatty alcohol, a fatty acid, a tenside, a fatty acid mono-, di- or triglyceride, a polyethylene glycol, a sugar, a wax, a fatty acid fatty alcohol ester, a sugar ester or a polyethylene glycol fatty acid ester or a polyethylene glycol fatty alcohol ether, and has a melt temperature above room temperature but below 200° C, a molecular weight of less than 20 000 Daltons and a melt viscosity of less than 100 Pa·s at the melt temperature of the acrylic plastic.

2. A thermoplastic coating and bonding agent according to claim 1, characterised in that the thermoplastic acrylic plastic A is a copolymerisate of 5 to 99 wt.-% alkyl esters of acrylic and/or methacrylic acid and 1 to 95 wt.-% aminoalkylesters or aminoalkylamides of acrylic and/or methacrylic acid or the salts or quaternary products thereof.

## Revendications

1. Agent thermoplastique d'enrobage et de liaison pour formes médicamenteuses, contenant un mélange inhomogène qui a été mélangé à une température de 100 à 150°C et qui a figé à partir de la masse fondue, constitué de :
A) 5 à 95 % en poids, par rapport à la somme du poids de A et de B, d'une matière synthétique acrylique thermoplastique, qui est un copolymère d'esters alkyliques de l'acide acrylique et/ou de l'acide méthacrylique et de comonomères fonctionnels avec des groupes cationiques liés de façon covalente, ayant une température de fusion supérieure à la température ambiante, mais inférieure à 200°C, une température de transition vitreuse inférieure à 120°C et une viscosité à l'état fondu de 1 000 à 1 million de Pa.s à la température de fusion, et
B) 95 à 5 % en poids d'un agent fluidifiant non compatible avec la matière plastique acrylique thermoplastique à la température ambiante, qui est un alcool gras, un acide gras, un agent tensioactif, un mono-, di- ou triglycéride d'acide gras, un polyéthylène glycol, un sucre, une cire, un ester d'alcool gras-acide gras, un ester de sucre ou un ester d'acide gras de polyéthylène glycol, ou un éther d'alcool gras de polyéthylène glycol, et qui a une température de fusion supérieure à la température ambiante, mais inférieure à 200°C, un poids moléculaire inférieur à 20 000 daltons et une viscosité à l'état fondu inférieure à 100 mPa.s à la température de fusion de la matière plastique acrylique.

2. Agent thermoplastique d'enrobage et de liaison selon la revendication 1,
caractérisé en ce que
la matière synthétique acrylique thermoplastique A est un copolymère de 5 à 99 % en poids d'esters alkyliques de l'acide acrylique et/ou de l'acide méthacrylique et de 1 à 95 % en poids d'esters aminoalkyliques ou d'aminoalkylamides de l'acide acrylique et/ou de l'acide méthacrylique ou de leurs sels ou produits de quaternisation.
